(19) Europäisches Patentamt
European Patent Office
Office européen des brevets

(11) Numéro de publication : 0 282 409 B1

(12) FASCICULE DE BREVET EUROPEEN

(45) Date de publication du fascicule du brevet : 19.06.91 Bulletin 91/25

(51) Int. Cl.[5] : C07C 29/136, C07C 31/08, C07C 31/125

(21) Numéro de dépôt : 88400544.8

(22) Date de dépôt : 08.03.88

# (54) Procédé de fabrication d'alcools par hydrogénolyse d'esters d'acides carboxyliques en présence d'un catalyseur contenant du ruthénium et de l'étain, du germanium ou du plomb.

(30) Priorité : 12.03.87 FR 8703525

(43) Date de publication de la demande : 14.09.88 Bulletin 88/37

(45) Mention de la délivrance du brevet : 19.06.91 Bulletin 91/25

(84) Etats contractants désignés : BE DE GB IT NL

(56) Documents cités :
EP-A- 0 095 408
EP-A- 0 128 059
EP-A- 0 172 091
DE-A- 3 019 582

(73) Titulaire : INSTITUT FRANCAIS DU PETROLE
4, Avenue de Bois-Préau
F-92502 Rueil-Malmaison (FR)

(72) Inventeur : Louessard, Pierrick
16, rue Villeroy
F-69003 Lyon (FR)
Inventeur : Candy, Jean-Pierre
4, chemin de la Mascotte
F-69300 Caluire (FR)
Inventeur : Mabilon, Gil
36, rue de Tourville
F-78100 Saint Germain en Laye (FR)
Inventeur : Bournonville, Jean-Paul
43, rue des Groues Vaureál
F-95000 Cergy Pontoise (FR)

EP 0 282 409 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

L'invention concerne un procédé catalytique de production d'alcools par hydrogénolyse d'esters d'acides carboxyliques.

La production d'alcools, et en particulier d'alcools gras, présente un intérêt considérable pour l'industrie.

L'hydrogénolyse catalytique des esters d'acides carboxyliques représente une voie intéressante de production de ces alcools mais elle s'est trouvée freinée jusqu'à présent par les inconvénients des catalyseurs connus :

– les catalyseurs à base d'oxydes mixtes de cuivre et de chrome, dopés ou non, nécessitent de travailler sous pression élevée, dans la quasi totalité des cas supérieure à 20 MPa, et à une température comprise entre 250 et 350°C,

– les catalyseurs à base de métaux de transition déposés sur un support obligent en général à travailler à une température moins élevée, inférieure à 250°C et de préférence à 200°C, pour limiter la dégradation de l'alcool formé en hydrocarbures ; ce qui impose des pressions opératoires supérieures à 10 MPa pour obtenir de bonnes sélectivités à un niveau de conversion acceptable.

Récemment on a proposé, dans le brevet US-A-4 456 775 un catalyseur formé par une association de rhodium avec, par exemple de l'étain, déposé sur un support comme par exemple la silice ou l'alumine. Ce catalyseur permet d'obtenir dans des conditions opératoires relativement douces (température inférieure à environ 280°C et pression inférieure à environ 8 MPa) de bons rendements en alcools. Néanmoins, le coût élevé et la disponibilité limitée du rhodium sont des inconvénients importants dont il faut tenir compte et qui ont conduit la demanderesse à poursuivre des recherches dans le but de trouver un catalyseur fournissant de bons rendements en alcools, permettant de travailler dans des conditions relativement douces et ne présentant pas les inconvénients liés à l'emploi du rhodium.

Postérieurement, dans le brevet US-A-4 628 130 on a décrit l'hydrogénation d'un ester en alcool en présence d'un catalyseur contenant du nickel et au moins un second élément choisi dans le groupe constitué de l'étain, du germanium et du plomb, résultant de l'incorporation d'au moins un composé d'au moins un métal du groupe Sn, Ge et Pb à un support contenant du nickel ou à du nickel de Raney.

Ce catalyseur contenant du nickel répond au souci de disponibilité et de coût des métaux actifs. Néanmoins l'utilisation de ces catalyseurs pour obtenir des conversions élevées nécessite souvent d'employer des températures relativement élevées. On constate alors que lorsque la température croit on obtient effectivement une augmentation de l'activité du catalyseur, mais ce gain s'accompagne d'une diminution relativement importante de la sélectivité en alcools.

Il a été découvert, de façon surprenante, que la sélectivité en alcool peut être maintenue a un niveau relativement élevé quand la température augmente si l' on utilise un catalyseur comprenant sur un support du ruthénium et au moins un second métal choisi dans le groupe constitué de l'étain, du germanium et du plomb.

Les esters d'acides carboxyliques qui peuvent être transformés en alcools sont avantageusement choisis dans le groupe des esters d'acides monocarboxyliques, par exemple les esters d'acides acétique, propionique, butyrique, valérique, caproïque, oléïque ou palmitique, ou polycarboxyliques en particulier dicarboxyliques comme par exemple les esters des acides oxalique, malonique ou adipique, avec des alcools alkyliques linéaires ou branchés tels que par exemple le méthanol ou l'éthanol, ou des alcools aralkyliques tels que par exemple l'alcool benzylique, ou des composés hydroxylés aromatiques par exemple le phénol ou des polyols tels que par exemple l'éthylène glycol. Il est également possible d'hydrogéner des polyesters ou des esters cycliques, en particulier les lactones, par exemple la valérolactone ou la caprolactone. L'hydrogénation d'un ester d'un monoacide carboxylique avec un monoalcool, sans modification de la structure de la chaine hydrocarbonée, est représentée par le schéma suivant :

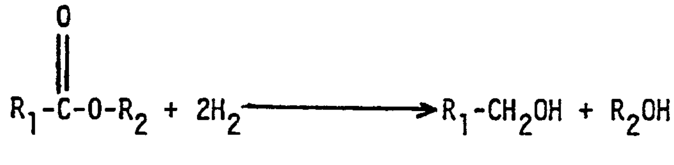

où $R_1$ représente habituellement un radical hydrocarbyl, saturé ou insaturé, de 1 à 30 atomes de carbone, par exemple un radical alkyl de $C_1$ à $C_{20}$, un radical aryl de $C_6$ à $C_{22}$ ou un radical aralkyl de $C_7$ à $C_{30}$ et $R_2$ représente habituellement un radical hydrocarbyl de $C_1$ à $C_{30}$, par exemple un radical aryl de $C_6$ à $C_{22}$, un radical aralkyl de $C_7$ à $C_{30}$, ou un radical alkyl de $C_1$ à $C_{20}$, et de préférence $R_2$ représente un radical alkyl de $C_1$ à $C_{10}$ et d'une façon particulièrement préférée méthyl ou éthyl.

On opère de préférence dans un réacteur continu ou discontinu sous une pression totale habituellement d'environ 10 à 100 bars (1 à 10 mégapascals) et de préférence d'environ 30 à 80 bars (3 à 8 MPa), bien que l'on puisse opérer sans inconvénient à des pressions plus élevées, par exemple jusqu'à 300 bars, à une température habituellement d'environ 180 à 330°C et de préférence d'environ 200 à 280°C et avec un rapport molaire hydrogène sur ester habituellement d'environ 2 : 1 à 50 : 1 et plus avantageusement d'environ 2 : 1 à 10 : 1, en présence d'un catalyseur métallique supporté renfermant les éléments suivants : le ruthénium dont le pourcentage pondéral est habituellement d'environ 0,1 à 5% et de préférence d'environ 0,5 à 3% et au moins un élément choisi dans le groupe constitué par le germanium, l'étain et le plomb et dont le pourcentage pondéral est habituellement d'environ 0,1 à 20% et plus particulièrement d'environ 1 à 12% et très avantageusement d'environ 2 à 5%. On peut avantageusement utiliser à la fois deux des métaux du groupe ci-dessus ou même les trois métaux de ce groupe ; le support peut être choisi dans le groupe constitué par la silice, les différents types d'alumine, les silices-alumines, les aluminates des éléments des groupes $I_A$, $II_A$ ou $II_B$ de la classification périodique des éléments (Hanbook of Chemistry and Physics 66ième edition 1985-86) comme par exemple les aluminates de Na, K, Ca, Mg, Ba, Zn, Cd, les aluminates mixtes, et le charbon. On emploie de préférence un support choisi dans le groupe constitué par la silice, les aluminates de métaux alcalins et/ou alcalino-terreux et/ou de zinc et/ou de cadmium et les aluminates mixtes.

Le catalyseur peut être préparé par différentes procédures d'imprégnation du support et l'invention n'est pas limitée à une procédure déterminée. L'opération d'imprégnation consiste, par exemple, à mettre en contact le support préformé et une solution aqueuse ou organique d'un composé du métal ou des métaux choisi(s), le volume de solution étant de préférence en excès par rapport au volume de rétention du support ou égal à ce volume. On peut introduire le ruthénium et le métal additionnel simultanément ou successivement. Après avoir laissé le contact entre le support et la solution pendant plusieurs heures, le support imprégné est filtré, lavé à l'eau distillée, séché et calciné sous air habituellement entre 110 et 600°C et de préférence entre 110 et 500°C. Avant utilisation, on réduit le catalyseur sous hydrogène habituellement entre 200 et 600°C et de préférence entre 300 et 500°C, cette réduction pouvant être effectuée aussitôt après calcination, ou plus tard, chez l'utilisateur.

L'élément choisi dans le groupe constitué par l'étain, le germanium et le plomb peut être introduit en solution aqueuse ou en solution hydrocarbonée selon la nature du précurseur utilisé.

D'une manière préférée le catalyseur est obtenu par imprégnation du support à l'aide d'une solution aqueuse ou organique d'au moins un composé de ruthénium, le volume de solution étant de préférence en excès par rapport au volume de rétention du support ou égal à ce volume. Le support imprégné est ensuite filtré, éventuellement lavé à l'eau distillée puis séché et calciné sous air habituellement entre environ 110°C et environ 600°C, et de préférence entre environ 110°C et environ 500°C, puis ensuite réduit sous hydrogène à une température habituellement comprise entre environ 200°C et environ 600°C et de préférence entre environ 300°C et environ 500°C ; le produit obtenu est alors imprégné par une solution aqueuse ou organique d'un composé de germanium, d'étain et/ou de plomb d'une manière particulièrement avantageuse on utilise une solution d'au moins un hydrocarbyl-germanium, un hydrocarbyl-étain ou un hydrocarbyl-plomb dans un hydrocarbure saturé.

Parmi les solvants organiques utilisables dans le cadre de l'invention on peut citer à titre d'exemples non limitatifs les hydrocarbures, les hydrocarbures halogénés, les cétones et les éthers. L'emploi d'un solvant n'est pas indispensable lorsque le composé de germanium, d'étain et/ou de plomb est lui-même liquide comme cela est par exemple le cas pour le tétrabutylétain.

Après avoir laissé le contact entre le support imprégné de ruthénium et la solution contenant au moins un composé de germanium, étain ou plomb pendant plusieurs heures, le produit est filtré, éventuellement lavé à l'aide du solvant employé pour déposer le germanium, l'étain et/ou le plomb, séché et éventuellement calciné sous air habituellement entre environ 110°C et environ 600°C, de préférence entre environ 110°C et 500°C. Avant utilisation on réduit le catalyseur sous hydrogène habituellement entre environ 200°C et environ 600°C et de préférence entre environ 300°C et environ 500°C, cette réduction pouvant être effectuée aussitôt après la calcination, ou plus tard chez l'utilisateur.

Une autre méthode consiste à malaxer la poudre humide de support avec les précurseurs du catalyseur et à mettre ensuite en forme et sécher.

Les exemples des précurseurs métalliques utilisables dans la préparation du catalyseur sont les suivants:

Pour le ruthénium, on peut employer des composés tels que par exemple les chlorures et les sels d'acides organiques solubles dans le solvant d'imprégnation.

A titre d'exemple non limitatif on peut citer : les chlorures de ruthénium, le chlororuthénate d'ammonium, le trichlorure de ruthénium hexammine, le dichlorure de ruthénium chloropentammine, l'acétate de ruthénium III, et le trioxalate de ruthénium III. On peut encore utiliser des composés organométalliques de ruthénium en solution dans un solvant organique, par exemple dans un hydrocarbure. Comme exemples d'hydrocarbures

on peut citer les hydrocarbures paraffiniques saturés dont la chaîne hydrocarbonée renferme de 6 à 12 atomes de carbone, les hydrocarbures naphténiques qui renferment de 6 à 12 atomes de carbone dans leur molécule ou encore les hydrocarbures aromatiques renfermant de 6 à 11 atomes de carbone dans leur molécule.

A titre d'exemple de composés organométalliques de ruthénium on peut citer : le triruthénium dodécacarbonyle, le diruthénium erméacarboxyle, les composés chlorocarbonyles du ruthénium et le triacétylacétonate de ruthénium.

L'élément choisi dans le groupe constitué de l'étain, du germanium et du plomb peut être introduit par l'intermédiaire de composés tels que les chlorures, les bromures et le nitrate d'étain, les halogénures, nitrate et acétate de plomb, le chlorure et l'oxalate de germanium en solution aqueuse ou organique ou, de préférence sous forme d'hydrocarbyl métaux tels que des alkyls ou des arylmétaux d'étain, de germanium et de plomb par exemple : le tétraéthyl-étain, le tétraméthyl-étain, le tétrapropyl-germanium, le tétraéthyl-plomb, le diphényl-étain, le diphényl-germanium, le tétraphényl-plomb avantageusement en solution hydrocarbonée.

Le support peut être de nature variée, comme déjà mentionné plus haut. Un support particulièrement adapté possède des caractéristiques spécifiques telles qu'une surface spécifique, déterminée par la méthode B.E.T., comprise entre 10 et 500 mètres carrés par gramme et de préférence comprise entre 50 et 500 mètres carrés par gramme et un volume poreux total de 0,2 à 1,3 $cm^3/g$ de support et de préférence de 0,5 à 1,1 $cm^3/g$ de support.

Une fois les métaux fixés sur le support, le catalyseur subit avantageusement un traitement d'activation sous hydrogène à haute température, par exemple 300-500°C, afin d'obtenir une phase métallique active. La procédure de ce traitement sous hydrogène consiste par exemple en une montée lente de la température sous courant d'hydrogène jusqu'à la température maximale de réduction, comprise par exemple entre 300 et 500°C et de préférence entre 350 et 450°C, suivie d'un maintien pendant par exemple 1 à 6 heures à cette température.

Les exemples suivants, non limitatifs, illustrent l'invention.

EXEMPLE 1 :

La préparation du catalyseur se fait en deux étapes :

– fixation du ruthénium par imprégnation de chlorure de ruthénium chloropentammine en solution ammoniacale sur une silice dont la surface spécifique est égale à 280 $m^2$ par gramme et le volume poreux total est égal à 80 $cm^3$ pour 100 grammes, suivies d'une filtration, d'un séchage à 110°C, d'une calcination sous air à 450°C et d'une réduction sous hydrogène à 450°C,

– fixation de l'étain sur le support préimprégné par le ruthénium calciné et réduit, sous forme de tétraéthylétain en solution dans le normal-heptane. Après avoir laissé en contact pendant 4 heures le support préimprégné par le ruthénium et la solution de tétraéthylétain au reflux de l'heptane, le catalyseur est lavé par l'heptane puis séché.

Le catalyseur est ensuite chargé dans un réacteur tubulaire puis réduit pendant 4 heures à 300°C sous courant d'hydrogène.

Les conditions opératoires pour l'hydrogénolyse de l'acétate d'éthyle sont les suivantes :

– pression : 50 bars (5,0 MPa)

– PPH : 4 kg/kg de catalyseur/h

– rapport molaire $H_2$/ester : 5.

Dans cette première série d'essais on a fait varier la teneur en étain des catalyseurs en partant d'un catalyseur de base contenant 1% en poids de ruthénium. La température de travail a été fixée à 250°C. Les résultats sont donnés dans le tableau 1 ci-après :

TABLEAU 1

| Ru (% poids) | Sn (% poids) | Conversion totale (% poids) | Sélectivité alcool (% poids) |
|---|---|---|---|
| 1 | 0 | 53 | 1,89 |
| 1 | 0,5 | 3,8 | 68,42 |
| 1 | 0,8 | 6,0 | 95,00 |
| 1 | 1,5 | 13,8 | 95,65 |
| 1 | 2,3 | 26,0 | 96,89 |
| 1 | 2,6 | 31,7 | 98,74 |
| 1 | 3,0 | 37,0 | 98,92 |
| 1 | 10,0 | 21,0 | 99,56 |

EXEMPLE 2 :

On compare, à différentes températures, les propriétés catalytiques, vis-à-vis de l'hydrogénolyse de l'acétate d'éthyle, de deux catalyseurs : l'un à base de 1,0% de ruthénium sur la silice de l'exemple 1, l'autre à base de 1% de ruthénium et 3,0% d'étain sur le même support de silice. Toutes les autres conditions sont identiques à celles de l'exemple 1. Les résultats figurent dans le tableau 2 ci-après.

TABLEAU 2

| Température (°C) | Catalyseur | | | |
|---|---|---|---|---|
| | 1 % Ru/$SiO_2$ | | 1 % Ru + 3 % Sn/$SiO_2$ | |
| | Conversion (% poids) | Sélectivité alcool (% poids) | Conversion (% poids) | Sélectivité alcool (% poids) |
| 200 | 20 | 90,00 | 13,2 | 99,24 |
| 220 | 18 | 33,33 | 21,8 | 99,64 |
| 250 | 53 | 1,89 | 37,0 | 98,92 |
| 280 | 99,9 | 0 | 48,5 | 98,14 |

Dans tout l'intervalle de température considéré, on observe que le catalyseur bimétallique ruthénium-étain

déposé sur silice est incomparablement plus sélectif pour la production d'alcool. La sélectivité en alcool obtenue avec le catalyseur bimétallique varie relativement peu lorsque la température augmente.

EXEMPLE 3 (comparatif)

On compare, à différentes températures, les propriétés catalytiques, vis à vis de l'hydrogénolyse de l'acétate d'éthyle d'un catalyseur comprenant 0,58% poids de nickel et 3% poids d'étain sur la silice de l'exemple 1. Ce catalyseur est obtenu en suivant la procédure décrite dans l'exemple 1 en remplaçant le composé de ruthénium par de l'acétate de nickel. Toutes les autres conditions sont identiques à celles de l'exemple 1. Les résultats figurent dans le tableau 3 ci-après.

TABLEAU 3

| Température (°C) | Catalyseur 0,58 % Ni + 3 % Sn/$SiO_2$ | |
|---|---|---|
| | Conversion (% poids) | Sélectivité alcool (% poids) |
| 200 | 10,6 | 99,29 |
| 220 | 16,9 | 99,57 |
| 250 | 28,0 | 97,06 |
| 280 | 36,5 | 93,94 |

EXEMPLE 4 (comparatif).

On compare à différentes températures, les propriétés catalytiques, vis à vis de l'hydrogénolyse de l'acétate d'éthyle, d'un catalyseur comprenant 1% poids de rhodium et 3% poids d'étain sur la silice de l'exemple 1. Ce catalyseur est obtenu en suivant la procédure décrite dans l'exemple 1 en remplaçant le composé de ruthénium en solution ammoniacale par le trichlorure de rhodium en solution aqueuse. Toutes les autres conditions sont identiques à celles de l'exemple 1. Les résultats figurent dans le tableau 4 ci-après.

TABLEAU 4

| Température (°C) | Catalyseur 1 % Rh + 3 % Sn/$SiO_2$ | |
|---|---|---|
| | Conversion (% poids) | Sélectivité alcool (% poids) |
| 200 | 2,3 | 99,5 |
| 220 | 5,6 | 99,3 |
| 250 | 11,1 | 96,3 |
| 280 | 24,8 | 93,8 |

EXEMPLE 5 :

On propose de fabriquer de l'éthanol à partir d'acétate d'éthyle dans des conditions identiques à celles adoptées dans l'exemple 1. Le catalyseur est à base de ruthénium (1%) déposé sur la silice de l'exemple 1 et d'un deuxième élément du groupe de l'étain, du germanium et du plomb.

Le germanium et le plomb sont imprégnés en solution hydrocarbonée (normal-heptane) respectivement sous forme de tétraéthyl-germanium et de tétraéthyl-plomb. Le catalyseur à l'étain a été décrit à l'exemple 1.

Les catalyseurs, ainsi préparés, sont mis en oeuvre de la même façon que dans l'exemple 1 (T : 250°C, P : 50 bars, PPH : 4, rapport molaire $H_2$/ester : 5).

Les résultats sont donnés dans le tableau 5 ci-après.

TABLEAU 5

| Ru (% poids) | 2ème métal (% poids) | Conversion (% poids) | Sélectivité alcools (% poids) |
|---|---|---|---|
| 1 | 0 | 53 | 1,89 |
| 1 | Sn = 3,0 | 37,0 | 98,92 |
| 1 | Ge = 1,8 | 36,6 | 98,86 |
| 1 | Pb = 5,2 | 36,8 | 98,64 |

L'étain peut donc être remplacé par le germanium et le plomb sans altération significative des propriétés catalytiques de la masse active.

EXEMPLE 6 :

On prépare selon la méthode décrite dans l'exemple 1, des catalyseurs à base de ruthénium et d'étain sur des supports à base d'aluminates de métaux alcalins, alcalino-terreux, de zinc ou de cadmium. Ces supports

ont des surfaces spécifiques comprises entre 80 et 150 mètres carré par gramme et des volumes poreux compris entre 50 et 100 $cm^3$ pour 100 grammes. Les pourcentages respectifs de ruthénium et d'étain sont donnés dans le tableau 6.

L'hydrogénolyse de l'acétate d'éthyle a été effectuée dans des conditions identiques à celles de l'exemple 1 si ce n'est que la température de réaction est portée à 280°C. Les résultats sont rassemblés dans le tableau 6 ci-après.

TABLEAU 6

| Support | Ru (% poids) | Sn (% poids) | Conversion (% poids) | Sélectivité alcool (% poids) |
|---|---|---|---|---|
| Aluminate de sodium | 1 | 3,1 | 47,8 | 97,70 |
| Aluminate de potassium | 1 | 3,2 | 47,5 | 98,31 |
| Aluminate de magnésium | 1 | 3,0 | 47,9 | 97,91 |
| Aluminate de calcium | 1 | 2,9 | 48,1 | 98,12 |
| Aluminate de barium | 1 | 3,1 | 48,0 | 98,33 |
| Aluminate de zinc | 1 | 3,0 | 47,5 | 98,52 |
| Aluminate de cadmium | 1 | 3,0 | 48,2 | 98,13 |
| Aluminate de Mg et Ca | 1 | 3,0 | 47,7 | 98,53 |
| Aluminate de Ca et Ba | 1 | 2,9 | 48,1 | 97,71 |
| Aluminate de Zn et Mg | 1 | 3,2 | 48,0 | 98,33 |

On peut donc avantageusement utiliser les aluminates de métaux alcalins, alcalino-terreux et/ou de zinc

et/ou de cadmium comme supports de catalyseur.

EXEMPLE 7 :

On se propose de fabriquer différents alcools à partir de divers esters en présence d'un catalyseur à base de ruthénium (1%) et d'étain (3%) déposés sur silice et dans des conditions opératoires identiques à celles de l'exemple 1.

Les esters utilisés sont les suivants :

- acétate de butyle secondaire,
- acétate d'amyle,
- acétate d'hexyle,
- caprate d'éthyle,
- palmitate de méthyle,
- oléate de méthyle.

Les résultats sont groupés dans le tableau 7 ci-après.

TABLEAU 7

| Substrat | Alcools obtenus | Conversion (% poids) | Sélectivité alcools (% poids) |
|---|---|---|---|
| Acétate de butyle secondaire | Ethanol et butanol-2 | 36,0 | 97,78 |
| Acétate d'amyle | Ethanol et alcool amylique | 34,3 | 97,67 |
| Acétate d'hexyle | Ethanol et hexanol-1 | 36,0 | 98,06 |
| Caprate d'éthyle | Décanol-1 et éthanol | 35,5 | 96,90 |
| Palmitate de méthyle | Héxadécanol-1 et méthanol | 33,0 | 97,27 |
| Oléate de méthyle | Octadécanol-1 et méthanol | 34,0 | 97,94 |

## Revendications

1. Procédé de fabrication d'alcools dans lequel un ester d'acide carboxylique est traité par l'hydrogène en présence d'un catalyseur caractérisé en ce que le catalyseur renferme un support, du ruthénium et au moins un élément du groupe formé par l'étain, le germanium et le plomb.

2. Procédé selon la revendication 1 dans lequel le catalyseur renferme de 0,1 à 5% en poids de ruthénium et de 0,1 à 20% en poids d'au moins un élément du groupe formé par l'étain, le germanium et le plomb.

3. Procédé selon la revendication 1 dans lequel le catalyseur renferme de 0,5 à 3% en poids de ruthénium et de 1 à 12% en poids d'au moins un élément du groupe formé par l'étain, le germanium et le plomb.

4. Procédé selon l'une des revendications 1 à 3 dans lequel le support présente une surface de 10 à 500 $m^2 \times g^{-1}$ et un volume poreux de 0,2 à 1,3 $cm^3 \times g^{-1}$.

5. Procédé selon l'une des revendications 1 à 4 dans lequel le support est la silice.

6. Procédé selon l'une des revendications 1 à 4 dans lequel le support est un aluminate d'un élément des groupes IA, IIA ou IIB de la classification périodique des éléments ou un aluminate mixte.

7. Procédé selon l'une des revendications 1 à 6 dans lequel le catalyseur est obtenu par imprégnation du support par une solution aqueuse ou organique d'au moins un composé du ruthénium, séchage du support imprégné suivi d'une calcination sous air à une température de 110°C à 600°C puis réduction sous hydrogène à une température de 200 à 600°C puis introduction d'au moins un élément du groupe formé par l'étain, le germanium et le plomb à l'aide d'au moins un composé dudit élément.

8. Procédé selon la revendication 7 dans lequel l'élément du groupe formé par l'étain, le germanium et le plomb est introduit à l'aide d'une solution dans un solvant organique d'un composé choisi dans le groupe formé par les hydrocarbylétain, les hydrocarbylgermanium et les hydrocarbylplomb.

9. Procédé de fabrication d'alcools selon l'une des revendications 1 à 8 dans lequel la pression est de 1 à 10 MPa et la température de 180 à 330°C.

10. Procédé de fabrication d'alcools selon l'une des revendications 1 à 9 dans lequel le rapport molaire hydrogène/ester est de 2 : 1 à 50 : 1.

## Claims

1. A process for manufacturing alcohols wherein a carboxylic acid ester is treated by hydrogen in the presence of a catalyst, characterized in that the catalyst comprises a support, ruthenium and at least one element from the group formed by tin, germanium and lead.

2. A process as claimed in claim 1 wherein the catalyst comprises 0.1 to 5% by weight of ruthenium and 0.1 to 20% by weight of at least one element from the group formed by tin, germanium and lead.

3. A process as claimed in claim 1 wherein the catalyst comprises 0.5 to 3% by weight of ruthenium and 1 to 12% by weight of at least one element from the group formed by tin, germanium and lead.

4. A process as claimed in any one of claims 1 to 3 wherein the surface of the support ranges from 10 to 500 $m^2 \times g^{-1}$ and its pore volume ranges from 0.2 to 1.3 $cm^3 \times g^{-1}$.

5. A process as claimed in any one of claims 1 to 4 wherein the support is silica.

6. A process as claimed in any one of claims 1 to 4 wherein the support is an aluminate of an element from groups IA, IIA or IIB of the periodic table of elements or a mixed aluminate.

7. A process as claimed in any one of claims 1 to 6 wherein the catalyst is obtained by impregnating the support with an aqueous or organic solution of at least one ruthenium compound, drying the impregnated support and calcining under air at a temperature ranging from 110°C to 600°C, then reducing under hydrogen at a temperature ranging from 200°C to 600°C, and introducing at least one element from the group formed by tin, germanium and lead by means of at least one compound of said element.

8. A process as claimed in claim 7 wherein the element from the group formed by tin, germanium and lead is introduced by means of a solution in an organic solvent of a compound selected from the group formed by the hydrocarbyltin, the hydrocarbylgermanium and the hydrocarbyllead.

9. A process for manufacturing alcohols as claimed in any one of claims 1 to 8 wherein the pressure ranges from 1 to 10 MPa and the temperature from 180 to 330°C.

10. A process for manufacturing alcohols as claimed in any one of claims 1 to 9 wherein the molar ratio hydrogen/ester ranges from 2 : 1 to 50 : 1.

## Ansprüche

1. Verfahren zur Herstellung von Alkoholen, bei dem ein Carbonsäureester mit Wasserstoff in Gegenwart eines Katalysators behandelt wird, dadurch gekennzeichnet, daß der Katalysator einen Ruthenium-Träger und mindestens ein Element der Gruppe aus Zinn ; Germanium und Blei enthält.

2. Verfahren nach Anspruch 1, bei dem der Katalysator 0,1 bis 5 Gew.-% Ruthenium und 0,1 bis 20 Gew.-% von mindestens einem Element der Gruppe aus Zinn, Germanium und Blei enthält.

3. Verfahren nach Anspruch 1, bei dem der Katalysator 0,5 bis 3 Gew.-% Ruthenium und 1 bis 12 Gew.-% von mindestens einem Element der Gruppe aus Zinn, Germanium und Blei enthält.

4. Verfahren nach einem der Ansprüche 1 bis 3, bei dem der Träger eine Oberfläche von 10 bis 500 $m^2 \times g^{-1}$ und ein Porenvolumen von 0,2 bis 1,3 $cm^3 \times g^{-1}$ aufweist.

5. Verfahren nach einem der Ansprüche 1 bis 4, bei dem der Träger Siliciumdioxid ist.

6. Verfahren nach einem der Ansprüche 1 bis 4, bei dem der Träger ein Aluminat eines Elements der Gruppen IA, IIA oder IIB des periodischen Systems der Elemente oder ein gemischtes Aluminat ist.

7. Verfahren nach einem der Ansprüche 1 bis 6, bei dem der Katalysator erhalten wird, indem der Träger mit einer wässrigen oder organischen Lösung mindestens eines Ruthenium-Gemisches imprägniert wird, der imprägnierte Träger einer Trocknung, gefolgt von einer Kalzinierung unter Luft bei einer Temperatur von 110°C bis 600°C, und dann einer Reduzierung unter Wasserstoff bei einer Temperatur von 200°C bis 600°C unterzogen wird, und dann mindestens ein Element der Gruppe aus Zinn, Germanium und Blei mit Hilfe mindestens eines Gemisches des Elementes eingebracht wird.

8. Verfahren nach Anspruch 7, bei dem das Element der Gruppe aus Zinn, Germanium und Blei mit Hilfe einer Lösung in einem organischen Lösungsmittel eines Gemisches der Gruppe aus Kohlenwasserstoff-Zinn, Kohlenwasserstoff-Germanium und Kohlenwasserstoff-Blei eingebracht wird.

9. Verfahren zur Herstellung von Alkoholen nach einem der Ansprüche 1 bis 8, bei dem der Druck 1 bis 10 MPa und die Temperatur 180 bis 330°C beträgt.

10. Verfahren zur Herstellung von Alkoholen nach einem der Ansprüche 1 bis 9, bei dem das Molverhältnis von Wasserstoff/Ester 2 : 1 bis 50 : 1 beträgt.